# EUROPEAN PATENT APPLICATION

(11) **EP 4 746 444 A1**
(43) Date of publication of application: **20.05.2026**
(21) Application number: 25175272.1
(22) Date of filing: 09.05.2025
(51) Int. Cl.: H04R 1/10, A61F 11/14

(54) **EAR CUP AND OVER-EAR ACTIVE NOISE CANCELLATION (ANC) HEADPHONE**

(30) Priority: 13.11.2024 CN 202411618299
(71) Applicant: Dongguan Shengyang Electronics Co., Ltd., Dongguan Guangdong (CN)
(72) Inventor: Wang, Jiangyi, Dongguan (CN)
(74) Representative: Chung, Hoi Kan

(57) **Abstract**

The present disclosure relates to the technical field of over-ear active noise cancellation (ANC) headphones, and provides an ear cup and an over-ear ANC headphone. The ear cup includes a sound-absorbing member and soft rubber. The soft rubber is provided on an outer surface of the sound-absorbing member and has a Shore hardness of 0 A to 95 A. A noise cancellation effect of the ear cup can be effectively improved by placing a layer of soft rubber with the Shore hardness of 0 A to 95 A on the outer surface of the sound-absorbing member.

## Description

### TECHNICAL FIELD

The present disclosure relates to the technical field of over-ear active noise cancellation (ANC) headphones, and in particular, to an ear cup and an over-ear ANC headphone.

### BACKGROUND

As a common type of earphones in the market, the over-ear ANC headphone typically includes a headband and two ear cups respectively mounted at two ends of the headband.

Currently, it is common to fill the ear cup with foam to achieve noise cancellation. However, there are a large number of pores inside the foam, and external sounds can penetrate through the pores, resulting in a poor noise cancellation effect.

### SUMMARY

An objective of the present disclosure is to provide an ear cup and an over-ear ANC headphone, aiming to solve a technical problem of a poor noise cancellation effect of an existing ear cup.

According to a first aspect, the present disclosure provides an ear cup, including a sound-absorbing member and soft rubber, where the soft rubber is provided on an outer surface of the sound-absorbing member and has a Shore hardness of 0 A to 95 A.

The ear cup provided in the present disclosure achieves the following beneficial effect: A noise cancellation effect of the ear cup can be effectively improved by placing a layer of soft rubber with the Shore hardness of 0 A to 95 A on the outer surface of the sound-absorbing member.

Optionally, the ear cup further includes a support member and an outer skin assembly, where the outer skin assembly is connected to the support member, and the outer skin assembly and the support member jointly wrap the sound-absorbing member and the soft rubber.

Optionally, the soft rubber is provided on an outer peripheral surface of the sound-absorbing member.

Optionally, the soft rubber is provided on a surface of the sound-absorbing member away from the support member.

Optionally, the soft rubber completely covers the outer peripheral surface of the sound-absorbing member.

Optionally, the soft rubber completely covers the surface of the sound-absorbing member away from the support member.

Optionally, the soft rubber is provided on an inner peripheral surface of the sound-absorbing member.

Optionally, the soft rubber is provided on a surface of the sound-absorbing member close to the support member.

Optionally, the soft rubber provided on the outer peripheral surface of the sound-absorbing member has a thickness of 0.5 mm to 2 mm.

Optionally, the soft rubber provided on the surface of the sound-absorbing member away from the support member has a thickness of 0.3 mm to 1 mm.

Optionally, the soft rubber provided on the inner peripheral surface of the sound-absorbing member has a thickness of 0.3 mm to 1 mm.

Optionally, the soft rubber provided on the surface of the sound-absorbing member close to the support member has a thickness of 0.3 mm to 1 mm.

Optionally, the sound-absorbing member is made of foam, and has an elliptical cross-section.

Optionally, the soft rubber is made of one of silica gel, gel, thermoplastic polyurethane (TPU), thermoplastic elastomer (TPE), thermoplastic rubber (TPR), polyvinyl chloride (PVC), and rubber.

According to a second aspect, the present disclosure provides an over-ear ANC headphone, including the above ear cup.

### BRIEF DESCRIPTION OF THE DRAWINGS

To describe the technical solutions in the embodiments of the present disclosure more clearly, the following briefly describes the accompanying drawings required for describing the embodiments or the prior art. Apparently, the accompanying drawings in the following description merely show some embodiments of the present disclosure, and persons of ordinary skill in the art may still derive other accompanying drawings from these accompanying drawings without creative efforts.
FIG. 1 is a cross-sectional view of an ear cup according to an embodiment of the present disclosure;
FIG. 2 is an enlarged view of A shown in FIG. 1;
FIG. 3 is a cross-sectional view of a sound-absorbing member and soft rubber (after explosion) according to an embodiment of the present disclosure;
FIG. 4 is an exploded view of an ear cup according to an embodiment of the present disclosure;
FIG. 5 illustrates a frequency response test (left ear cup) according to an embodiment of the present disclosure; and
FIG. 6 illustrates a frequency response test (right ear cup) according to an embodiment of the present disclosure.

### Reference numerals in the figures:

100: ear cup; 11: support member; 12: sound-absorbing member;
13: soft rubber; 14: protein leather; 15: polyurethane (PU) leather;
16: connecting member; 200: outer skin assembly

### DETAILED DESCRIPTION OF THE EMBODIMENTS

The embodiments of the present disclosure are described below in detail. Examples of the embodiments are shown in the drawings. The same or similar numerals represent the same or similar elements or elements having the same or similar functions throughout the specification. The embodiments described below with reference to the accompanying drawings are exemplary, and are intended to explain the present disclosure but should not be construed as a limitation to the present disclosure.

The term "one embodiment" or "embodiment" referred to in the description means that a specific feature, structure or characteristic described in connection with the embodiments is included in at least one embodiment of the present disclosure. Thus, the statements "in one embodiment" or "in some embodiments" in the specification do not necessarily refer to a same embodiment. In addition, the specific features, structures or characteristics may be appropriately combined in one or more embodiments of the present disclosure.

In the description of the present disclosure, it should be understood that orientations or position relationships indicated by terms "length", "width", "upper", "lower", "front", "rear", "left", "right", "vertical", "horizontal", "top", "bottom", "inside", "outside", and the like are based on the orientations or position relationships shown in the accompanying drawings. These terms are just used to facilitate the description of the present disclosure and simplify the description, but not to indicate or imply that the mentioned device or elements must have a specific orientation and must be established and operated in a specific orientation, and thus these terms cannot be understood as a limitation to the present disclosure.

In addition, the terms "first" and "second" are merely intended for a purpose of description, and shall not be understood as an indication or implication of relative importance or implicit indication of a quantity of indicated technical features. Thus, features defined with "first" and "second" may explicitly or implicitly include one or more of the features.

In the present disclosure, unless otherwise clearly specified, the terms "installation", "interconnection", "connection" and "fixation" etc. are intended to be understood in a broad sense. For example, the "connection" may be a fixed connection, removable connection or integral connection; may be a mechanical connection or electrical connection; may be a direct connection or indirect connection using a medium; and may be a communication or interaction between two elements. Those of ordinary skill in the art may understand specific meanings of the above terms in the present disclosure based on a specific situation.

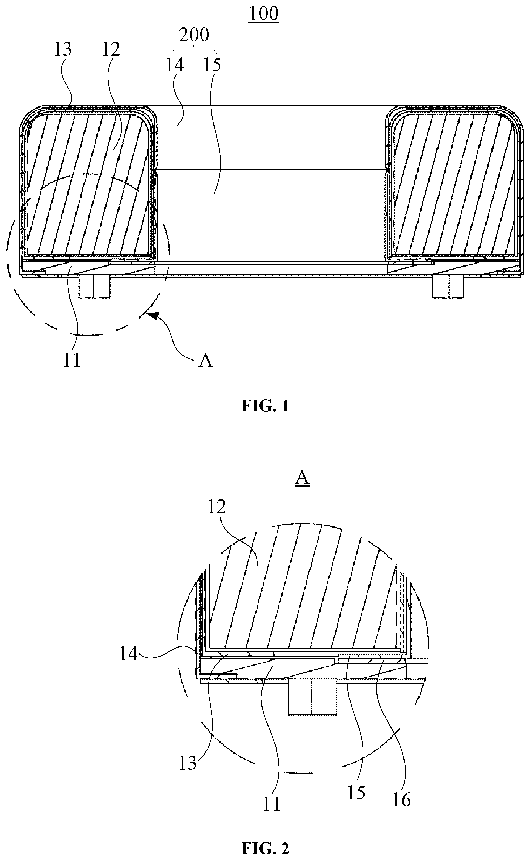

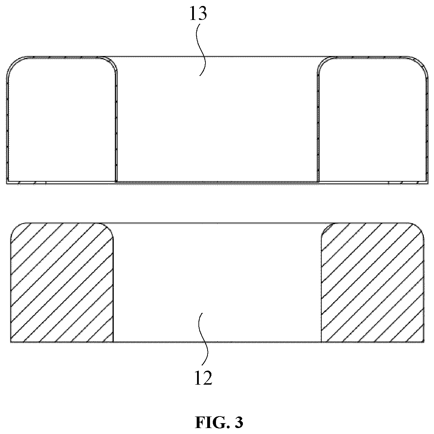

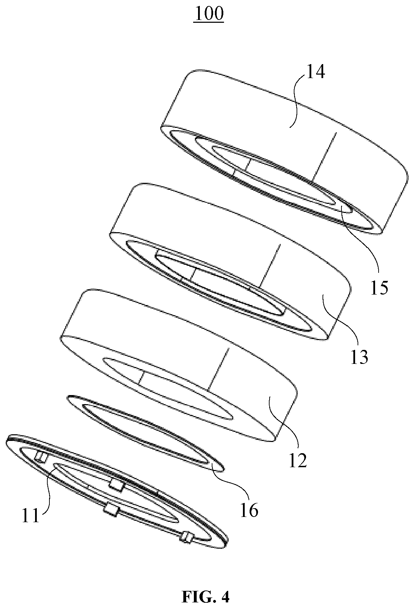

Referring to FIG. 1 to FIG. 6, an ear cup 100 and an over-ear ANC headphone in the embodiments of the present disclosure are described below.

Referring to FIG. 1 to FIG. 4, the ear cup 100 provided in the present disclosure includes a sound-absorbing member 12 and soft rubber 13. The soft rubber 13 is provided on an outer surface of the sound-absorbing member 12 and has a Shore hardness of 0 A to 95 A. Specifically, the sound-absorbing member 12 is made of foam, and is preferably made of memory foam. In addition, the sound-absorbing member 12 has an elliptical cross-section, and a support member 11 is of a hollow structure. In the prior art, most ear cups 100 are only filled with the foam inside. Due to a large number of pores inside the foam, a noise cancellation effect of such an ear cup 100 is not ideal. In the present disclosure, a layer of soft rubber 13 with the Shore hardness of 0 A to 50 A is provided on an outer surface of the foam, which can effectively improve the noise cancellation effect of the ear cup 100.

As an example, the Shore hardness of the soft rubber 13 may be 0 A, 5 A, 10 A, 15 A, 20 A, 25 A, 30 A, 35 A, 40 A, 45 A, 50 A, 55 A, 60 A, 65 A, 70 A, 75 A, 80 A, 85 A, 90 A, 95 A, or a value between any two of the aforementioned values. A user's comfort can be improved by controlling the Shore hardness of the soft rubber 13 within the above range.

The Shore hardness of the soft rubber in the present disclosure is measured as follows: a soft rubber sample with an area (L×W) of 5 cm² and a thickness of 5-10 mm is selected and used as a test piece; the test piece is tested using a Type A Shore hardness tester according to the ASTM D-2240 test method, to obtain the Shore hardness of the soft rubber.

In some embodiments, referring to FIG. 1, the ear cup 100 further includes the support member 11 and an outer skin assembly 200. The outer skin assembly 200 is connected to the support member 11, and the outer skin assembly 200 and the support member 11 jointly wrap the sound-absorbing member 12 and the soft rubber 13. Specifically, the support member 11 is a rubber member.

In some embodiments, referring to FIG. 1 to FIG. 4, the soft rubber 13 is provided on an outer peripheral surface of the sound-absorbing member 12.

In some embodiments, referring to FIG. 1 to FIG. 4, the soft rubber 13 is provided on a surface of the sound-absorbing member 12 away from the support member 11.

In some embodiments, referring to FIG. 1 to FIG. 4, the soft rubber 13 is provided on both the outer peripheral surface of the sound-absorbing member 12 and the surface of the sound-absorbing member 12 away from the support member 11.

The above three arrangement methods of the soft rubber 13 can all improve the noise cancellation effect of the ear cup 100. When the soft rubber 13 is provided on both the outer peripheral surface of the sound-absorbing member 12 and the surface of the sound-absorbing member 12 away from the support member 11, the ear cup 100 achieves an optimal noise cancellation effect.

In some embodiments, referring to FIG. 1 to FIG. 4, the soft rubber 13 completely covers the outer peripheral surface of the sound-absorbing member 12. This setting can further improve the noise cancellation effect of the ear cup 100.

In some embodiments, referring to FIG. 1 to FIG. 4, the soft rubber 13 completely covers the surface of the sound-absorbing member 12 away from the support member 11. This setting can further improve the noise cancellation effect of the ear cup 100.

In some embodiments, referring to FIG. 1 to FIG. 4, the soft rubber 13 is provided on an inner peripheral surface of the sound-absorbing member 12 to achieve a sealing function.

In some embodiments, referring to FIG. 1 to FIG. 4, the soft rubber 13 is provided on a surface of the sound-absorbing member 12 close to the support member 11 to achieve the sealing function.

In some embodiments, the soft rubber 13 is formed through injection molding and sleeved on the sound-absorbing member 12.

In some embodiments, the soft rubber 13 provided on the outer peripheral surface of the sound-absorbing member 12 has a thickness of 0.5 mm to 2 mm. As an example, the thickness of the soft rubber 13 provided on the outer peripheral surface of the sound-absorbing member 12 may be 0.5 mm, 0.6 mm, 0.7 mm, 0.8 mm, 0.9 mm, 1.0 mm, 1.1 mm, 1.2 mm, 1.3 mm, 1.4 mm, 1.5 mm, 1.6 mm, 1.7 mm, 1.8 mm, 1.9 mm, 2 mm, or a value between any two of the aforementioned values, and is preferably 1 mm.

In some embodiments, the soft rubber 13 provided on the surface of the sound-absorbing member 12 away from the support member 11 has a thickness of 0.3 mm to 1 mm. As an example, the thickness of the soft rubber 13 provided on the surface of the sound-absorbing member 12 away from the support member 11 may be 0.3 mm, 0.4 mm, 0.5 mm, 0.6 mm, 0.7 mm, 0.8 mm, 0.9 mm, 1 mm, or a value between any two of the aforementioned values, and is preferably 0.5 mm.

In some embodiments, the soft rubber 13 provided on the inner peripheral surface of the sound-absorbing member 12 has a thickness of 0.3 mm to 1 mm. As an example, the thickness of the soft rubber 13 provided on the inner peripheral surface of the sound-absorbing member 12 may be 0.3 mm, 0.4 mm, 0.5 mm, 0.6 mm, 0.7 mm, 0.8 mm, 0.9 mm, 1 mm, or a value between any two of the aforementioned values, and is preferably 0.5 mm.

In some embodiments, the soft rubber 13 provided on the surface of the sound-absorbing member 12 close to the support member 11 has a thickness of 0.3 mm to 1 mm. As an example, the thickness of the soft rubber 13 provided on the surface of the sound-absorbing member 12 close to the support member 11 may be 0.3 mm, 0.4 mm, 0.5 mm, 0.6 mm, 0.7 mm, 0.8 mm, 0.9 mm, 1 mm, or a value between any two of the aforementioned values, and is preferably 0.5 mm.

The thickness of the soft rubber 13 on each part of the sound-absorbing member 12 is controlled within the above range, which can ensure the noise cancellation effect of the ear cup 100 while reducing a cost.

In some embodiments, the soft rubber is made of one of silica gel, gel, TPU, TPE, TPR, PVC, and rubber.

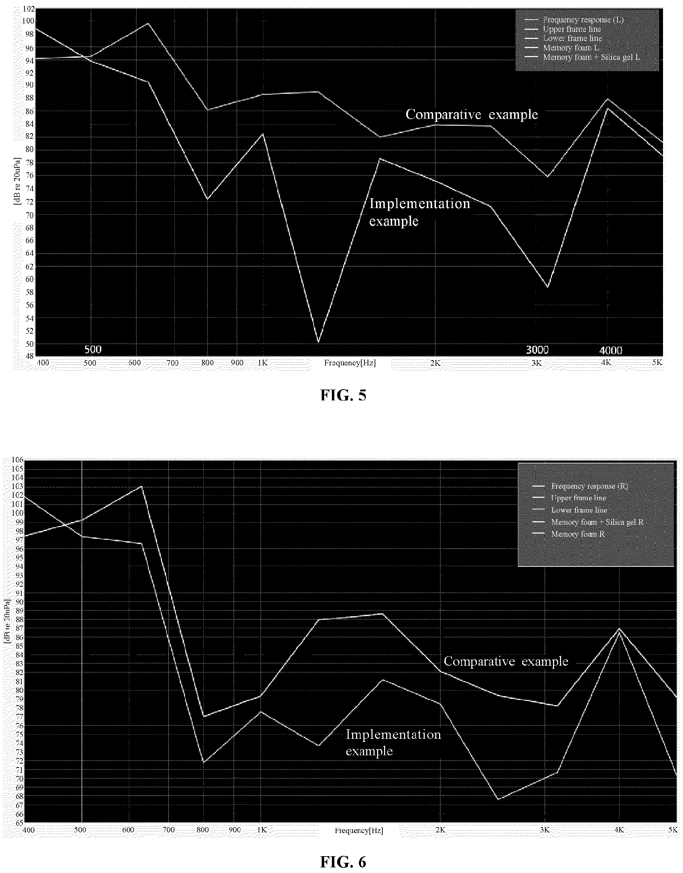

Referring to FIG. 5 and FIG. 6, in a frequency response test chart, a horizontal axis represents a frequency in units of Hz, and a vertical axis represents sound intensity in units of dB. A comparative example is an ear cup that has only memory foam, and an implementation example is the following ear cup: The soft rubber 13 is provided on both the outer peripheral surface of the sound-absorbing member 12 and the surface of the sound-absorbing member 12 away from the support member 11, the soft rubber 13 completely covers the outer peripheral surface of the sound-absorbing member 12, and the soft rubber 13 completely covers the surface of the sound-absorbing member 12 away from the support member 11. The thickness of the soft rubber provided on the outer peripheral surface of the sound-absorbing member is 1 mm, and the thickness of the soft rubber provided on the surface of the sound-absorbing member away from the support member is 0.5 mm. In a frequency range of 500 Hz to 3000 Hz, sound intensity of the implementation example is less than that of the comparative example, which means that the ear cup in the implementation example has a better noise cancellation effect than an ear cup in the prior art.

In some embodiments, referring to FIG. 1 and FIG. 2, the outer skin assembly 200 includes protein leather 14 and polyurethane (PU) leather 15. The protein leather 14 covers a part of a surface of the support member 11 away from the sound-absorbing member 12, the outer peripheral surface of the sound-absorbing member 12, the surface of the sound-absorbing member 12 away from the support member 11, and a part of the inner peripheral surface of the sound-absorbing member 12. The PU leather 15 covers a part of the inner peripheral surface of the sound-absorbing member 12 and a part of the surface of the sound-absorbing member 12 close to the support member 11. One end of the protein leather 14 is connected to the PU leather 15, and the other end of the protein leather 14 is connected to the support member 11. One end of the PU leather 15 is connected to the protein leather 14, and the other end of the PU leather 15 is connected to the support member 11. The protein leather 14 is beneficial for improving the user's comfort. The PU leather 15 is easy to make and convenient to produce.

In some embodiments, referring to FIG. 2, the PU leather 15 is hot-pressed with a connecting member 16. The connecting member 16 is made of polyethylene terephthalate (PET) and bonded with the support member 11. The PU leather 15 is difficult to bond with the support member 11 and is prone to wrinkling. The connecting member 16 is relatively hard and easy to bond with the support member 11. The connecting member 16 facilitates a connection between the PU leather 15 and the support member 11.

In the frequency range of 500 Hz to 3000 Hz, passive noise cancellation of the ear cup 100 provided in the present disclosure is 5 dB to15 dB lower than that of the existing ear cup filled only with the memory foam.

The present disclosure further provides an over-ear ANC headphone, including the above ear cup 100.

The above are merely preferred examples of the present disclosure, and not intended to limit the present disclosure. Any modifications, equivalent replacements, and improvements made within the spirit and principle of the present disclosure should fall within the protection scope of the present disclosure.

## Claims

1. An ear cup, comprising:
a sound-absorbing member (12); and
soft rubber (13) provided on an outer surface of the sound-absorbing member (12), wherein the soft rubber (13) has a Shore hardness of 0 A to 95 A.

2. The ear cup according to claim 1, further comprising a support member (11) and an outer skin assembly (200), wherein the outer skin assembly (200) is connected to the support member (11), and the outer skin assembly (200) and the support member (11) jointly wrap the sound-absorbing member (12) and the soft rubber (13).

3. The ear cup according to claim 2, wherein
the soft rubber (13) is provided on an outer peripheral surface of the sound-absorbing member (12); and/or
the soft rubber (13) is provided on a surface of the sound-absorbing member (12) away from the support member (11).

4. The ear cup according to claim 3, wherein
the soft rubber (13) completely covers the outer peripheral surface of the sound-absorbing member (12); and/or
the soft rubber (13) completely covers the surface of the sound-absorbing member (12) away from the support member (11).

5. The ear cup according to claim 3, wherein
the soft rubber (13) is provided on an inner peripheral surface of the sound-absorbing member (12); and/or
the soft rubber (13) is provided on a surface of the sound-absorbing member (12) close to the support member (11).

6. The ear cup according to claim 3, wherein
the soft rubber (13) provided on the outer peripheral surface of the sound-absorbing member (12) has a thickness of 0.5 mm to 2 mm; and/or
the soft rubber (13) provided on the surface of the sound-absorbing member (12) away from the support member (11) has a thickness of 0.3 mm to 1 mm.

7. The ear cup according to claim 5, wherein
the soft rubber (13) provided on the inner peripheral surface of the sound-absorbing member (12) has a thickness of 0.3 mm to 1 mm; and/or
the soft rubber (13) provided on the surface of the sound-absorbing member (12) close to the support member (11) has a thickness of 0.3 mm to 1 mm.

8. The ear cup according to any one of claims 1 to 7, wherein the sound-absorbing member (12) is made of foam, and has an elliptical cross-section.

9. The ear cup according to any one of claims 1 to 7, wherein the soft rubber (13) is made of one of silica gel, gel, thermoplastic polyurethane (TPU), thermoplastic elastomer (TPE), thermoplastic rubber (TPR), polyvinyl chloride (PVC), and rubber.

10. An over-ear active noise cancellation (ANC) headphone, comprising the ear cup (100) according to any one of claims 1 to 9.
